(19) Europäisches Patentamt European Patent Office Office européen des brevets

(11) EP 3 881 240 B1

(12) FASCICULE DE BREVET EUROPEEN

(45) Date de publication et mention
de la délivrance du brevet:
**02.04.2025  Bulletin 2025/14**

(21) Numéro de dépôt: **19801894.7**

(22) Date de dépôt: **18.11.2019**

(51) Classification Internationale des Brevets (IPC):
*G06N 3/02* (2006.01)    *G06N 20/10* (2019.01)
*A61B 34/10* (2016.01)

(52) Classification Coopérative des Brevets (CPC):
**G06N 20/00;** A61B 2034/107

(86) Numéro de dépôt international:
**PCT/EP2019/081571**

(87) Numéro de publication internationale:
**WO 2020/099671 (22.05.2020 Gazette 2020/21)**

(54) **METHODE DE DETERMINATION D'UNE CIBLE CEREBRALE STEREOTAXIQUE**

VERFAHREN ZUR BESTIMMUNG EINES STEREOTAKTISCHEN HIRNTARGETS

METHOD FOR DETERMINING A STEREOTACTIC BRAIN TARGET

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **16.11.2018  FR 1860588**

(43) Date de publication de la demande:
**22.09.2021  Bulletin 2021/38**

(73) Titulaires:
• **UNIVERSITE DE BORDEAUX**
  **33000 Bordeaux (FR)**
• **Institut Polytechnique de Bordeaux**
  **33402 Talence Cedex (FR)**
• **Centre Hospitalier Universitaire de Bordeaux**
  **33404 Talence (FR)**
• **Centre national de la recherche scientifique**
  **75016 Paris (FR)**
• **Institut National de Recherche en
  Informatique et en Automatique (INRIA)**
  **78153 Le Chesnay Cedex (FR)**

(72) Inventeurs:
• **ENGELHARDT, Julien**
  **33140 Villenave D'Ornon (FR)**
• **ZEMZEMI, Nejib**
  **33800 Bordeaux (FR)**
• **CUNY, Emmanuel**
  **33000 Bordeaux (FR)**

(74) Mandataire: **Atout PI Laplace
Immeuble Up On
25 Boulevard Romain Rolland
CS 40072
75685 Paris Cedex 14 (FR)**

(56) Documents cités:
**WO-A2-2018/008034    US-A1- 2014 171 791**

• JINYOUNG KIM ET AL: "Automatic Localization
of the Subthalamic Nucleus on Patient-Specific
Clinical MRI by Incorporating 7T MRI and
Machine Learning: Application in Deep Brain
Stimulation", BIORXIV, 15 May 2018 (2018-05-15),
XP055614540, Retrieved from the Internet
<URL:https://www.biorxiv.org/content/biorxiv/
early/2018/05/15/322230.full-text.pdf> [retrieved
on 20190822], DOI: 10.1101/322230
• FRANÇOIS CAIRE: "Imagerie per-opératoire des
électrodes de stimulation cérébrale profonde et
proposition d'une nouvelle modalité de repérage
stéréotaxique indirect de la cible
subthalamique", 20 December 2012 (2012-12-20),
XP055614390, Retrieved from the Internet
<URL:https://pdfs.semanticscholar.org/7b48/
b5adcd69491fe6224655a791c98956a74d28.pdf?
_g
a=2.168037729.1525806837.1566385871-4830773
91.1566385871> [retrieved on 20190821]

## Description

DOMAINE TECHNIQUE DE L'INVENTION

L'invention se situe dans le domaine de la neurochirurgie, et concerne plus précisément une méthode de préparation avant une intervention neurochirurgicale.

L'invention concerne plus particulièrement une méthode de détermination précise d'une cible cérébrale stéréotaxique.

L'invention peut concerner en particulier une méthode de détermination d'une cible cérébrale à stimuler en tant qu'étape de préparation avant l'implantation d'une électrode de stimulation cérébrale profonde.

ETAT DE LA TECHNIQUE

La détermination d'une cible cérébrale est essentielle dans tout protocole de traitement neurochirurgical, en particulier en stéréotaxie. La stéréotaxie est une technique de neurochirurgie qui utilise une méthode de repérage tridimensionnel des structures intracrâniennes assistée par l'imagerie médicale afin de déterminer avec précision, à partir d'un point situé à l'intérieur du cerveau, le volume et la localisation d'une région du cerveau ou d'une lésion sur laquelle le neurochirurgien doit intervenir.

Une intervention peut être la stimulation cérébrale profonde (« SCP » ou « DBS » en anglais pour « Deep Brain Stimulation »). La stimulation cérébrale profonde est une technique chirurgicale très efficace pour soulager les patients atteints de pathologies neurologiques telles que la maladie de Parkinson, la maladie du tremblement essentiel, la dystonie, le trouble obsessionnel compulsif, le syndrome Gille de la Tourette, l'épilepsie réfractaire, voire la dépression sévère et résistante.

La stimulation cérébrale profonde consiste en la stimulation d'une structure profonde et hyperactive du cerveau. La stimulation peut inhiber ou activer les neurones, l'objectif étant de moduler le fonctionnement des réseaux neuronaux. Elle peut réduire voire supprimer les symptômes et améliorer la qualité de vie des patients résistants au traitement habituel.

La stimulation cérébrale profonde est généralement réalisée en implantant des électrodes sur une structure cérébrale qui correspond à un noyau, et généralement plutôt une partie de noyau de substance grise (corps cellulaires des neurones). Par la suite de la présente description, le terme « noyau » pourra nommer indifféremment un noyau ou une partie de noyau.

Les électrodes reçoivent un courant électrique de faible intensité qui est ainsi délivré au noyau visé.

Les structures cérébrales visées dans la stimulation cérébrale profonde varient en fonction des pathologies à traiter : le noyau sous-thalamique (« NST » ou « STN » en anglais pour « SubThalamic Nucleus ») pour traiter la maladie de Parkinson, le noyau ventral intermédiaire du thalamus (« VIM ») pour traiter le tremblement essentiel, le Globus Pallidus interne (GPi) pour traiter la dystonie et certaines formes de maladie de Parkinson. Les dimensions de ces noyaux n'excèdent pas quelques millimètres mis à part le Globus Pallidus interne.

Parmi les défis que représente cette technique, il y a l'identification (c'est-à-dire la définition et/ou la localisation) du noyau à stimuler, le repérage (c'est à dire le ciblage) du noyau identifié et le positionnement des électrodes de stimulation.

Le premier défi concerne l'identification du noyau à stimuler. Par exemple, en ce qui concerne le VIM, il existe plusieurs nomenclatures du thalamus humain, ce qui a pour conséquence des controverses sur l'identification du VIM. Les problèmes de définition anatomique du STN sont moins importants que pour le VIM. Il subsiste en revanche des controverses quant à la sous-partie du STN devant être stimulée, voire si les fibres situées au-dessus du STN (« zona incerta ») ne constituent pas plutôt la meilleure cible.

Quand bien même ce problème d'identification était résolu, il reste les autres défis que sont le repérage du noyau identifié et donc le positionnement des électrodes de stimulation. Par exemple, si le noyau GPi pour traiter la dystonie est repérable à l'aide d'une Imagerie par Résonance Magnétique (« IRM »), le noyau STN et le noyau VIM sont beaucoup moins repérables voire impossibles à repérer à l'aide d'une IRM utilisable en pratique stéréotaxique courante (typiquement une IRM avec un champ magnétique à 1,5 ou 3 Tesla). Ainsi, le repérage de certains noyaux est certes amélioré grâce aux progrès des IRM : par exemple, une combinaison de séquences IRM permet de visualiser le noyau STN (séquences 3D T1, T2, SWI (« Susceptibily Weighted Imaging »), FGATIR (« Fast Grey matter AcquisiTion Inversion Recovery »), FLAIR (« Fluid Attenuation Inversion Recovery »)). Cependant, il a été démontré que le noyau STN visualisé radiologiquement par IRM ne correspond pas exactement au noyau STN pour laquelle la stimulation fonctionne (ce qui peut être mesuré par la technique d'enregistrement électrophysiologique peropératoire décrite plus après), des erreurs de plus 5 mm pouvant se voir à l'échelle individuelle.

Pour ces zones moins visibles ou non repérables, il est connu de mettre en œuvre des techniques de repérage dit « indirect » basées sur des atlas stéréotaxiques. Le principe de ces techniques de repérage indirect est de disposer d'un référentiel cartésien comprenant des points de repérage anatomiques facilement identifiables par une IRM, ainsi que d'un atlas indiquant la localisation de différentes zones cérébrales (par exemple différents noyaux du cerveau) qui ne sont pas toutes repérables par une IRM. Le principe est de repérer la position de n'importe quelle zone dans le cerveau d'un individu quelconque à partir de tout ou partie des points de repérage en utilisant cet atlas. On utilise un ensemble de fonctions de proportionnalité entre les points de repérage et les points de l'atlas permettant ainsi de réaligner tout cerveau, indépendamment de sa taille ou de ses spécificités anatomiques.

Les atlas stéréotaxiques sont utilisés plus largement lorsque le cerveau est étudié, bien au-delà de l'application à la SCP. Un exemple connu est l'atlas ou référentiel de Talairach. Le centre de ce référentiel (i.e. le point de coordonnées x=0, y=0, z=0) est le bord supérieur et postérieur de la commissure antérieure dans le plan médio-sagittal (i.e. le plan parallèle à la face interne du cerveau aussi appelé plan inter-hémisphérique), ce point nommé « CA » (également nommé « AC ») est très facilement visible en IRM. Un autre point de référence est utilisé : le bord inférieur et antérieur de la commissure postérieure (toujours dans le plan médio-sagittal), ce point nommé « CP » (également nommé « PC ») est également très facilement visible en IRM. Les trois axes du repères sont alors définis de la façon suivante : l'axe Oy passe par CA et CP et est orienté vers l'avant du crâne. L'axe Oz est l'axe du plan sagittal perpendiculaire à Oy et passant par CA. Il est orienté vers le sommet du crâne. Enfin l'axe Ox est l'axe orthogonal à Oy et à Oz passant par CA, il est orienté de gauche à droite. Un autre exemple est l'atlas de Schaltenbrand qui a permis de simplifier le système de Talairach en prenant comme centre du référentiel stéréotaxique le milieu du segment [CACP], appelé point mi-commissural. Les points CA et CP représentent respectivement le centre anatomique des commissures antérieure et postérieure.

Une fois le noyau à stimuler identifié en fonction de la pathologie à traiter, on le repère dans l'atlas utilisé à l'aide des points de repérage identifiables par IRM. Lors de la stimulation profonde, l'électrode de stimulation devra être positionnée sur le noyau ainsi indirectement repéré.

Le problème des techniques de repérage indirect connues est qu'elles sont basées sur l'hypothèse de la proportionnalité du cerveau, comme si la variabilité de la distance entre deux points pouvait rendre compte de toutes les variabilités anatomiques observées, ce qui n'est pas le cas. Cela induit donc nécessairement des imprécisions de ciblage qui peuvent être très préjudiciables en stimulation cérébrale profonde.

Pour pallier ces imprécisions de ciblage, la chirurgie actuelle ne peut se passer d'enregistrements électrophysiologiques et de tests cliniques peropératoires afin de s'assurer que l'électrode est bien positionnée dans la cible. Le principe de l'enregistrement électrophysiologique peropératoire est d'implanter des microélectrodes parallèles au niveau de la région supposée contenir la cible anatomique, de stimuler les neurones de la région et d'enregistrer en peropératoire l'effet de la stimulation. Cette technique permet d'optimiser la position de l'électrode définitive et d'affiner ainsi le ciblage. Si cette technique permet d'améliorer la précision de ciblage, elle est néanmoins très longue (durée d'opération de l'ordre de 7 à 10 heures), et elle entraîne des risques infectieux, une augmentation du risque hémorragique proportionnelle au nombre de microélectrodes d'enregistrement utilisées. En outre, l'implantation de l'électrode définitive n'est pas toujours aisée à réaliser de manière précise. Enfin, l'opération chirurgicale nécessite la participation du patient afin de pouvoir tester l'effet clinique de la stimulation et ainsi optimiser la position de l'électrode. Elle doit donc être réalisée sous anesthésie locale, ce qui est très inconfortable pour le patient.

Les techniques susmentionnées sont basées sur une approche consistant en la définition d'un noyau à stimuler, puis au ciblage dudit noyau, puis au positionnement d'une électrode de stimulation dans le cas d'une SCP. L'analyse du résultat obtenu peut aboutir au report de la position de l'électrode de stimulation et/ou au recalage de la cible anatomique dans le référentiel utilisé (généralement le référentiel de Schaltenbrand ou de Talairach).

Une autre technique consiste en une approche indirecte consistant en l'identification de patients pour lesquels la stimulation cérébrale profonde a été concluante, la récupération de la position des électrodes chez ces patients et la corrélation avec des points référentiels facilement identifiables sur une IRM, puis le report chez de nouveaux patients à traiter en utilisant les points référentiels. Cette approche permet de ne pas multiplier les facteurs d'imprécisions que sont l'identification du noyau à stimuler, le repérage (le ciblage) de la cible et le positionnement des électrodes de stimulation au niveau de la cible. On pourra parler dans ce cas de « cible clinique » ou de « cible fonctionnelle », étant donné que la cible de stimulation est déterminée à partir de cas cliniques qui ont fonctionné.

Une telle approche est décrite dans le document de thèse de François Caire *« Imagerie per-opératoire des électrodes de stimulation cérébrale profonde et proposition d'une nouvelle modalité de repérage stéréotaxique indirect de la cible subthalamique »*. La solution proposée dans cette thèse comprend les étapes suivantes :

- sélection de patients présentant une très bonne réponse à la stimulation profonde subthalamique ;
- récupération des IRM postopératoires desdits patients ;
- construction pour chaque IRM postopératoire de l'espace stéréotaxique standard CACP : pour cela, les points CA et CP et le plan inter-hémisphérique sont identifiés ;
- détermination sur les IRM postopératoires des coordonnées du contact actif de simulation ;
- détermination sur les IRM postopératoires de différents points de repères anatomiques :
- calcul de la corrélation entre chaque coordonnée du contact actif et la coordonnée correspondante de chaque point de repère, en utilisant un modèle de régression linéaire ;

- lorsque la corrélation est correcte, calcul de l'équation de la droite de régression à partir des points de repère qui offrent la meilleure corrélation avec les coordonnées x, y et z des contacts actifs ;
- calcul des coordonnées d'une cible théorique en utilisant cette équation ;
- comparaison des coordonnées de cette cible théorique aux coordonnées effectives des contacts actifs.

L'inconvénient de cette méthode est qu'elle fait l'hypothèse de la variabilité (ou de l'homothétie) du cerveau sur seulement 3 axes, en restant dans un système de proportionnalité (ici une méthode de régression linéaire). Cela est mieux que de prendre l'hypothèse que la variabilité de la distance entre deux points (CA et CP) peut rendre compte de toutes les variabilités anatomiques observées, mais cela reste néanmoins insuffisant. Ainsi, l'erreur moyenne de la méthode est de $2.5 \pm 0.6$ mm, ce qui est assez important en comparaison de la taille des structures à cibler.

L'invention vise à surmonter les inconvénients précités de l'art antérieur.

Plus particulièrement l'invention vise à disposer d'une méthode de détermination d'une cible cérébrale stéréotaxique qui permette une meilleure précision de ciblage, et qui soit aisée à utiliser. Elle vise à disposer d'une méthode de détermination d'une cible cérébrale stéréotaxique utilisée en tant qu'étape de préparation avant un traitement neurochirurgical qui permette d'améliorer l'efficacité d'un tel traitement.

EXPOSE DE L'INVENTION

Un objet de l'invention permettant d'atteindre ce but est une méthode de détermination d'une cible cérébrale stéréotaxique comprenant au moins un point de ciblage, ladite méthode comprenant les étapes suivantes :

- sélection de patients pour lesquels le résultat mesuré suite à un traitement en au moins un point de ciblage est supérieur ou égal à un seuil, une imagerie postopératoire ayant été réalisée pour chacun desdits patients ;
- traitement de ladite imagerie postopératoire de manière à déterminer pour chaque patient sélectionné toute ou partie des coordonnées du au moins un point de ciblage ;
- sélection de points de repérages du cerveau ;
- traitement de ladite imagerie postopératoire de manière à déterminer pour chaque patient sélectionné toute ou partie des coordonnées des points de repérage sélectionnés ;
- constitution d'une base d'apprentissage comprenant les coordonnées des points de ciblage et les coordonnées des points de repérages déterminés pour l'ensemble des patients sélectionnés ;
- détermination d'une fonction de prédiction donnant les coordonnées d'au moins un point de ciblage en fonction des coordonnées des points de repérages en utilisant la base d'apprentissage et une méthode d'apprentissage statistique supervisé ;
- traitement d'une imagerie préopératoire d'un nouveau patient de manière à déterminer toute ou partie des coordonnées des points de repérages dudit nouveau patient ;
- utilisation de la fonction de prédiction de manière à obtenir les coordonnées d'au moins un point de ciblage pour ledit nouveau patient en fonction des coordonnées des points de repérages déterminés pour ledit nouveau patient.

De manière préférée, l'invention concerne une méthode de détermination d'une cible cérébrale stéréotaxique comprenant au moins un point de ciblage, ladite méthode étant préalable à un traitement neurochirurgical au niveau de ladite cible pour une pathologie donnée et comprenant les étapes suivantes :

- sélection d'une pluralité de cas cliniques de patients pour lesquels le résultat mesuré suite au traitement réalisé pour ladite pathologie en au moins un point de ciblage est supérieur ou égal à un seuil, une imagerie postopératoire ayant été réalisée pour chacun desdits patients ;
- sélection d'un repère mathématique, de préférence un repère cartésien orthonormé ;
- traitement de ladite imagerie postopératoire de manière à déterminer pour chaque cas clinique sélectionné toute ou partie des coordonnées du au moins un point de ciblage dans le repère sélectionné ;
- sélection d'une pluralité de points de repérages du cerveau ;
- traitement de ladite imagerie postopératoire de manière à déterminer pour chaque cas clinique sélectionné toute ou partie des coordonnées des points de repérage du cerveau ;
- constitution d'une base d'apprentissage comprenant les coordonnées déterminées des points de ciblage et les coordonnées déterminées des points de repérages de l'ensemble des cas cliniques sélectionnés ;
- détermination d'une fonction de prédiction donnant les coordonnées d'au moins un point de ciblage en fonction des points de repérages en utilisant la base d'apprentissage et une méthode d'apprentissage statistique supervisé ;
- traitement d'une imagerie préopératoire d'un nouveau patient à traiter pour ladite pathologie de manière à déterminer toute ou partie des coordonnées des points de repérages dudit nouveau patient ;

- utilisation de la fonction de prédiction de manière à obtenir les coordonnées d'au moins un point de ciblage pour ledit nouveau patient en fonction des coordonnées des points de repérages déterminés pour ledit nouveau patient.

Selon l'invention, les points de repérage sont des points caractéristiques de structures anatomiques du cerveau repérables dans un repère mathématique donné, et sont des points anatomiques visibles notamment à l'aide d'imagerie classique.

Selon l'invention, les points de ciblage sont des points sur lequel le traitement neurochirurgical a été appliqué pour les cas cliniques, ou doit être appliqué pour les nouveaux patients.

La méthode selon l'invention se base sur des données de cas cliniques de patients pour lesquels l'efficacité du traitement a été déterminée. Ainsi, les points de ciblage sur lesquels l'apprentissage est réalisé sont des cibles validées cliniquement. Ceci permet d'améliorer le traitement neurochirurgical qui peut suivre la méthode selon l'invention.

Pour ce faire, la méthode selon l'invention est basée sur de l'imagerie postopératoire disponible pour ces patients, laquelle imagerie est traitée pour en déduire : les points sur lesquels le traitement a été appliqué (points de ciblage) et des points de repérage choisis parmi des points caractéristiques de structures anatomiques du cerveau visibles notamment à l'aide d'une imagerie classique. Les coordonnées de ces points de ciblage et ces points de repérages sont intégrées dans une base d'apprentissage qui permet de déterminer une fonction de prédiction. La fonction de prédiction permet de donner au moins un point de ciblage en fonction de points de repérage. La fonction de prédiction est construite à partir de la base d'apprentissage en utilisant une méthode d'apprentissage statistique supervisé.

La méthode selon l'invention peut se baser sur de l'imagerie extraite d'une IRM classiquement utilisée, par exemple d'une IRM à 1.5 T. Il n'est pas nécessaire de disposer d'une IRM plus puissante, par exemple à 3T ou à 7T qui est beaucoup plus coûteuse (notamment en termes Hardware), et qui nécessite des compétences de radiologies plus importantes que celle à 1.5T. Ces images sont traitées afin de déterminer les coordonnées des points de repérage.

La méthode selon l'invention cherche à contrôler la variabilité des cerveaux de manière multidimensionnelle et non plus sur un, deux ou trois axes. En optant pour une technique d'apprentissage statistique supervisé, la méthode permet de s'affranchir de cette variabilité des cerveaux, et elle permet ainsi une meilleure précision de ciblage.

En outre, les points de ciblage sont déterminés en tant que cibles validées cliniquement, contrairement à d'autres méthodes où on cherche d'abord à identifier puis à repérer la structure cérébrale à stimuler (STN, VIM ...), puis à implanter l'électrode de stimulation dans la structure cérébrale ainsi identifiée et repérée. Ainsi, cela permet ne pas multiplier les facteurs d'imprécisions et donc d'améliorer la précision de ciblage.

En ce sens, la méthode selon l'invention ne cherche pas à construire un atlas anatomique pour visualiser les structures cérébrales (STN, VIM ...) en fonction de points de repérage, mais elle recherche des points de ciblage pour savoir directement où appliquer le traitement, et ce, en s'appuyant sur des points de ciblage qui ont fonctionné pour des patients. Les inventeurs ont en effet identifié que, même en repérant le centre de la structure anatomique (STN, VIM ...), rien ne garantit que ce centre soit la bonne cible à stimuler, comme cela est expliqué plus avant.

La méthode selon l'invention permet en outre de déterminer une cible (plusieurs points) et non seulement un point, et de construire ainsi une cible en 3D.

Une fois la cible déterminée pour un nouveau patient, il est possible de marquer la position de la cible dans l'IRM par un signe, par exemple une croix. Pour marquer à l'aide d'une croix, il est possible de changer la valeur de cinq pixels dans chacune des 3 directions du repère mathématique en affectant la valeur maximale (qui correspond à la couleur blanche), à ces pixels. Cela permet de former une croix blanche, le centre de cette croix blanche permet au praticien de visualiser la cible.

Enfin, la méthode de détermination d'une cible stéréotaxique selon l'invention peut être utilisée comme aide à la décision, notamment en préparation à tout traitement neurochirurgical nécessitant la détermination d'au moins une cible cérébrale précise.

En particulier, dans le cas de la stimulation cérébrale profonde, la méthode de l'invention permet de s'affranchir de l'électrophysiologie qui est généralement utilisée pour s'assurer que l'électrode est bien positionnée dans la structure cérébrale à stimuler. De ce fait, la méthode de la présente invention n'est pas invasive pour le patient.

En utilisant des techniques adaptées de sélection et d'optimisation des paramètres de la fonction de prédiction (également nommée « metamodèle »), la méthode permet d'améliorer encore davantage la précision de ciblage.

Ainsi, selon un mode de réalisation avantageux, la méthode peut comprendre en outre une étape de :

- consolidation de la fonction de prédiction en utilisant une méthode de validation croisée, ladite étape de consolidation aboutissant à une fonction de prédiction consolidée donnant les coordonnées d'au moins un point de ciblage en fonction des points de repérage ;

l'étape d'utilisation de la fonction de prédiction consistant en l'utilisation de la fonction de prédiction consolidée.

Selon un mode de réalisation, l'imagerie traitée pour déterminer les coordonnées des points de repérage et du au moins un point de ciblage est au moins une image IRM, de préférence plusieurs images IRM.

La méthode peut ainsi comprendre une étape de réalisation d'IRM postopératoire chez les patients sélectionnés et la détermination des points de repérage peut comprendre une étape de traitement des images obtenues par IRM et/ou la détermination du au moins un point de ciblage peut également comprendre une étape de traitement des images obtenues par IRM.

La méthode peut alternativement ou de manière complémentaire comprendre une étape de réalisation d'une tomodensitométrie (TDM) postopératoire chez les patients sélectionnés et la détermination du au moins un point de ciblage peut comprendre une étape de traitement des images obtenues par TDM et/ou la détermination des points de repérage peut comprendre une étape de traitement des images obtenues par TDM.

Selon un mode de réalisation, la méthode d'apprentissage statistique supervisé comprend l'utilisation d'une méthode de régression ridge à noyaux dans un espace de Hilbert à noyau reproduisant.

Selon un autre mode de réalisation, la méthode d'apprentissage statistique supervisé comprend l'utilisation d'une méthode de type Machine à Vecteurs de Supports.

Selon un autre mode de réalisation, la méthode d'apprentissage statistique supervisé comprend l'utilisation d'une méthode de type réseaux de neurones.

Selon un mode de réalisation, la méthode de validation croisée comprend l'utilisation d'une méthode « leave-one-out cross-validation ».

Selon un autre mode de réalisation, la méthode de validation croisée comprend l'utilisation d'une méthode « leave-k-out cross validation ».

Selon un mode de réalisation particulier, le repère mathématique choisi est un repère cartésien orthonormé, la droite passant par le bord supérieur et postérieur de la commissure antérieure et le bord inférieur et antérieur de la commissure postérieure formant l'axe Oy, le bord inférieur et antérieur de la commissure postérieure formant le centre du repère, et l'axe Oz étant la droite perpendiculaire à l'axe Oy dans le plan inter-hémisphérique.

Selon un mode de réalisation particulier, les points de repérage sont choisis parmi les dix-huit points suivants, de préférence définis par rapport au repère mathématique précédemment choisi :

- le premier point de repérage étant le faisceau mamillo-thalamique sur le troisième plan axial ;
- les deuxième, troisième et quatrième points de repérage étant la pointe antérieure du putamen sur chacun des premier, second et troisième plans axiaux ;
- les cinquième et sixième points de repérage étant la pointe médiale du putamen sur les premier et second plans axiaux ;
- les septième et huitième points de repérage étant la pointe postérieure du putamen sur les premier et second plans axiaux
- le neuvième point de repérage étant la commissure habénulaire sur le second plan axial ;
- le dixième point de repérage étant le bord antérieur du thalamus sur le second plan axial ;
- le onzième point de repérage étant le bord postérieur du thalamus sur le second plan axial ;
- le douzième point de repérage étant la commissure antérieure ;
- le treizième point de repérage étant le bord médial du troisième ventricule au point mi-commissural ;
- le quatorzième point de repérage étant la hauteur du thalamus sur le plan sagittal passant par le treizième point de repérage ;
- le quinzième point de repérage étant le milieu du segment défini par les treizième et quatorzième points de repérage ;
- le seizième point de repérage étant le bord antérieur du thalamus sur la droite parallèle à la droite passant par le bord supérieur et postérieur de la commissure antérieure et le bord inférieur et antérieur de la commissure postérieure et passant par le quinzième point de repérage ;
- le dix-septième point de repérage étant le bord supérieur du putamen sur le plan coronal passant par le cinquième point de repérage ; et
- le dix-huitième point de repérage étant le bord latéral du putamen sur le plan coronal passant par le cinquième point de repérage.

Selon un mode de réalisation, l'ensemble de ces dix-huit points de repérages sont utilisés.

D'autres points de repérage peuvent être utilisés.

D'autres repères mathématiques, notamment les repères habituellement utilisés dans les atlas stéréotaxiques connus, peuvent être utilisés.

Selon un mode de réalisation, la méthode comprend une étape supplémentaire d'ajout de données fonctionnelles dans la base d'apprentissage, lesdites données fonctionnelles étant aptes à ajouter au moins un indice du degré de confiance dans les points de ciblage et les points de repérages des cas cliniques.

Selon un mode de réalisation, la méthode est mise en œuvre préalablement à une stimulation cérébrale profonde. Selon un autre mode de réalisation la méthode est mise en œuvre préalablement à un gammaknife. Selon un autre mode de réalisation, la méthode est mise en œuvre préalablement à un traitement par ultrasons focalisés.

Les points de repérage, ou les points de ciblage sont adaptés en fonction du traitement prévu, et donc en fonction de la pathologie à traiter.

Un second objet de l'invention est un système de traitement de données comprenant un processeur configuré pour mettre en œuvre tout ou partie des étapes de la méthode. Cela peut être un ordinateur, une tablette, un smartphone...

Un troisième objet de l'invention est un programme d'ordinateur comprenant des instructions qui, lorsque le programme est exécuté par un processeur, conduisent celui-ci à mettre en œuvre tout ou partie des étapes de la méthode.

DESCRIPTION DES FIGURES

D'autres caractéristiques et avantages de l'invention apparaîtront à l'aide de la description qui suit donnée à titre illustratif et non limitatif, faite en regard des figures annexées parmi lesquelles :

- la figure 1 est une image de synthèse illustrant l'implantation de deux électrodes de stimulation en deux points différents et selon deux directions différentes ;
- la figure 2 est une image de synthèse illustrant une électrode implantée dans une cible et comprenant plusieurs contacts actifs ;
- les figures 3A à 3F sont des images IRM vues selon différentes coupes illustrant 18 points de repérage ;
- les figures 4A et 4C sont trois images IRM illustrant les axes du repère mathématique CA-CP et la figure 4D montre le repère mathématique CA-CP en 3D ;
- la figure 5 est un logigramme représentant un exemple de méthode selon l'invention, ainsi que des variantes.

EXPOSÉ DÉTAILLÉ DE MODES DE RÉALISATION PARTICULIERS

L'invention consiste à déterminer des points de ciblage pour un traitement neurochirurgical, lesquels points de ciblage sont positionnés généralement dans des structures profondes intracérébrales non visibles par imagerie classique.

Pour cela, l'invention consiste en l'exploitation de points de repérage visibles par imagerie classique et d'une fonction de prédiction. Ladite fonction de prédiction est construite en utilisant une base d'apprentissage comprenant des points de repérage et des points de ciblage provenant de cas cliniques de patients pour lesquels l'efficacité du traitement a été constatée. L'efficacité est déterminée par au moins un résultat mesuré comme expliqué plus après. Une technique d'apprentissage statistique supervisé est utilisée pour construire ladite fonction de prédiction (ou metamodèle), qui est ainsi apte à fournir les coordonnées d'au moins un point de ciblage en fonction de points de repérages.

La description des modes de réalisation qui suit est généralement illustrée dans le cas où la méthode selon l'invention est mise en œuvre en tant que préparation à un traitement par stimulation cérébrale profonde, étant entendu que l'invention et notamment lesdits modes de réalisation peuvent être mise en œuvre plus généralement en tant que préparation à tout traitement neurochirurgical nécessitant la détermination d'au moins une cible cérébrale précise.

Dans l'application particulière à la stimulation cérébrale profonde, un point de ciblage peut également être nommé point de stimulation.

Sélection des cas cliniques (patients) :

Les patients pour lesquels la stimulation par électrode en au moins un point de stimulation a été efficace pour traiter une même pathologie sont sélectionnés. L'efficacité est déterminée par au moins un résultat mesuré.

La pathologie peut être la maladie de Parkinson, la maladie du tremblement essentiel, la dystonie, le trouble obsessionnel compulsif, le syndrome Gille de la Tourette, l'épilepsie réfractaire, voire la dépression sévère et résistante.

La structure visée pour la stimulation peut être le noyau sous-thalamique (STN) pour traiter la maladie de Parkinson, le noyau ventral intermédiaire du thalamus (VIM) pour traiter le tremblement essentiel, le Globus Pallidus interne (GPi) pour traiter la dystonie et certaines formes de maladie de Parkinson.

Ce résultat mesuré suite au traitement neurochirurgical peut être une évaluation clinique postopératoire, une évaluation par localisation anatomique ou radiologique, ou une évaluation électrophysiologique.

Une évaluation clinique postopératoire est réalisée au moyen d'échelles spécifiques à la pathologie traitée et/ou d'échelles de qualité de vie, par exemple trois mois après le traitement. Cela permet de déterminer un premier critère et donc un premier seuil pour la sélection des cas cliniques (patients).

Une évaluation par localisation anatomique ou radiologique est définie par un ciblage préopératoire anatomique ou radiologique et une distance entre ledit ciblage préopératoire et le point de traitement en postopératoire (par exemple la position de l'électrode dans le cas de la SCP). Cela permet de déterminer un deuxième critère et donc un deuxième seuil pour la sélection des cas cliniques (patients).

Une évaluation électrophysiologique est définie par une identification peropératoire de la cible et la distance entre ladite identification peropératoire et le point de traitement en postopératoire (par exemple la position de l'électrode dans le cas de la SCP). Cela permet de déterminer un troisième critère et donc un troisième seuil pour la sélection des cas cliniques (patients).

Les différents critères d'évaluation peuvent être combinés pour définir une valeur de seuil combinée.

Les patients sélectionnés vont permettre de construire une base d'apprentissage statistique comprenant des couples points de repérage / points de ciblage pour au moins une pathologie.

En outre, la méthode peut comprendre une étape d'ajout de données fonctionnelles dans la base d'apprentissage des patients. Ces données peuvent comprendre les paramètres d'évaluation des symptômes de la pathologie en préopératoire et le degré d'amélioration des symptômes de la pathologie en postopératoire. On pourra prendre à titre d'exemple l'échelle UPDRS (Unified Parkinson Disease Rating Scale) pour la maladie de Parkinson ou l'échelle de Fahn-Tolosa et Marin pour le tremblement essentiel.

En outre, dans le cas de la stimulation cérébrale profonde, ces données peuvent comprendre les paramètres électriques (amplitude, fréquences et durées) de la stimulation.

Toutes ces données fonctionnelles peuvent ainsi être introduites dans la base d'apprentissage et dans le metamodèle, en plus des points de ciblage et des points de repérages. Tout ou partie de ces données fonctionnelles peuvent être utilisées dans une fonction coût comme au moins un indice de la confiance dans les données des patients. Dans ce cas, ces données sont traduites mathématiquement dans le metamodèle comme des fonctions poids qui vont pondérer la fonction coût à minimiser.

Les points de repérage sont des points caractéristiques de structures anatomiques du cerveau repérables dans le repère mathématique utilisé, et sont des points anatomiques visibles à l'aide d'une imagerie classique, notamment d'une IRM.

Ainsi, les points de repérage peuvent être obtenus en exploitant une imagerie issue d'IRM postopératoires existantes.

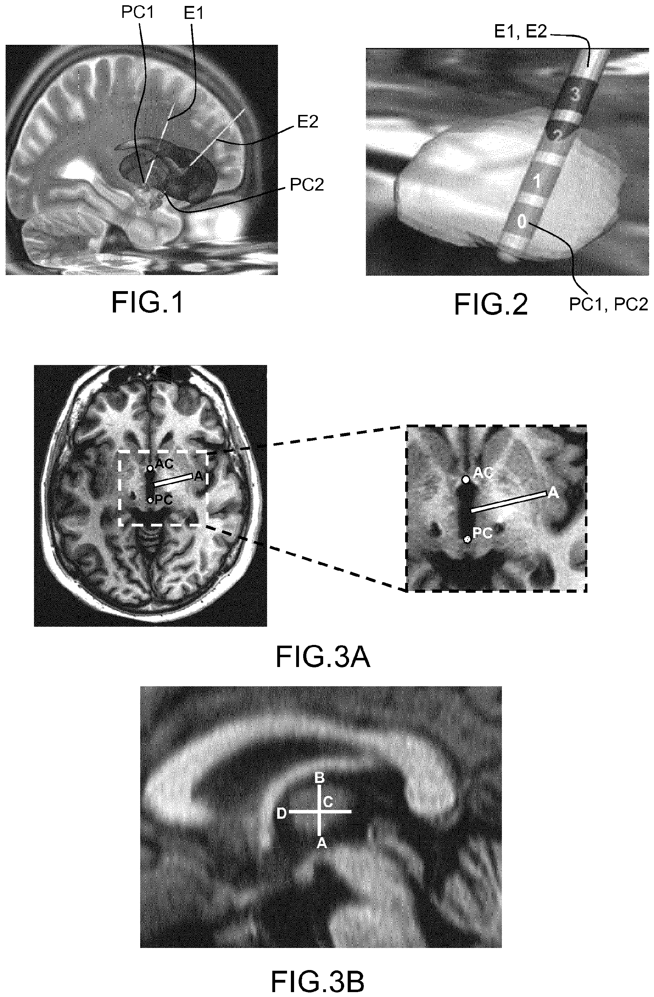

Dans le mode de réalisation particulier décrit, un point de ciblage (ou point de stimulation) est un point d'implantation de l'électrode. Un point de stimulation est caractérisé par ses coordonnées dans le repère mathématique utilisé. Il peut également être caractérisé par la direction d'une électrode (E1, E2) implantée en ce point (PC1, PC2), comme illustré sur les figures 1 et 2.

Chaque point de stimulation est déterminé par exemple en réalisant et en exploitant une imagerie post-opératoire, par exemple par IRM ou par tomodensitométrie (« TDM ») et en reconstruisant l'électrode dans le référentiel à partir de l'artéfact distal et des artéfacts créés par les différents contacts.

Dans la présente description, un point de ciblage ou l'ensemble de points de ciblage constitue une « cible », ou « cible de stimulation » dans l'application particulière de la stimulation cérébrale profonde. Une cible est caractérisée par les coordonnées de chaque point de ciblage. Dans l'application particulière de la stimulation cérébrale profonde, elle peut en outre être caractérisée par la direction de l'électrode en un point de stimulation (coordonnées du vecteur directeur de l'électrode).

Ces points de repérage et de ciblage déterminés pour les patients sélectionnés constituent ainsi une base d'apprentissage statistique, qui peut être enrichie par d'autres données.

La méthode selon l'invention comprend l'utilisation d'une méthode de régression basée sur l'apprentissage statistique supervisé, exploitant ladite base d'apprentissage, de manière à établir une fonction de prédiction entre les points de repérage et le(s) point(s) de ciblage.

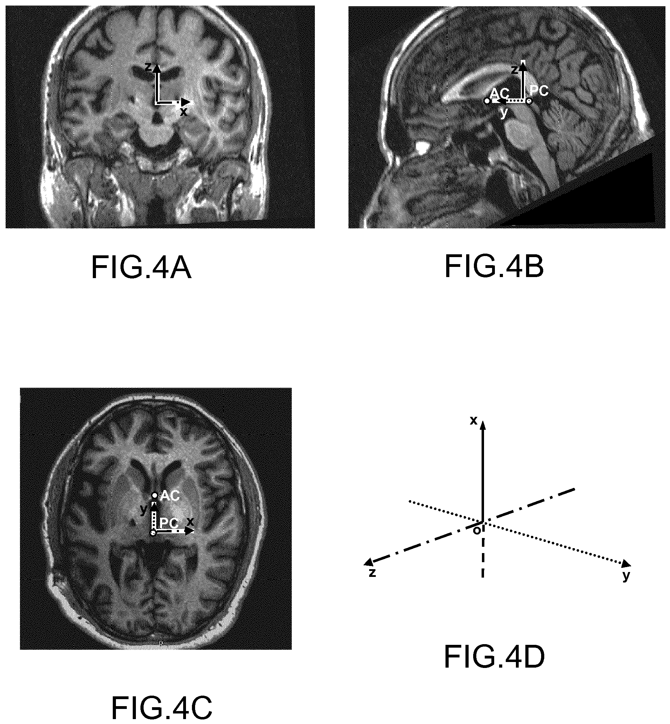

La description qui suit, donnée notamment en référence aux figures 3A à 3F et 4A à 4D décrit un mode de réalisation particulier de la méthode.

**Etape de construction et/ou d'initialisation de la base d'apprentissage**

Repère mathématique utilisé

On désigne par « CA » le bord supérieur et postérieur de la commissure antérieure et par « CP » le bord inférieur et antérieur de la commissure postérieure.

Le repère mathématique utilisé est illustré en figures 4A à 4D. La figure 4A est une coupe coronale, la figure 4B est une coupe sagittale médiane et la figure 4C est une coupe axiale. La figure 4D est une vue 3D illustrant les trois axes.

Le repère illustré est un repère cartésien orthonormé ayant comme origine le point CP et dont l'axe Oy est la droite passant par les points CA et CP (symbolisée « CACP »). L'axe Oz est la droite perpendiculaire à la droite (CACP) dans le plan inter-hémisphérique et l'axe Ox est déduit. Un tel repère est symbolisé dans la présente description par CA-CP.

Points de repérage utilisables

Dans un exemple de réalisation, les points de repérage utilisables sont au nombre de 18 (m = 18) correspondant aux points notamment représentés sur les figures 3A à 3F. Les dix-huit points de repérages utilisables sont :

- premier point de repérage (FMT) : le faisceau mamillo-thalamique sur le troisième plan axial ;
- deuxième, troisième et quatrième points de repérage (PA1, PA2, PA3) : la pointe antérieure du putamen sur chacun des premier, second et troisième plans axiaux ;
- cinquième et sixième points de repérage (PM1, PM2) : la pointe médiale du putamen sur les premier et second plans axiaux ;
- septième et huitième points de repérage (PP1, PP2) : la pointe postérieure du putamen sur les premier et second plans axiaux
- neuvième point de repérage (CH) : la commissure habénulaire sur le second plan axial ;
- dixième point de repérage (BAT) : le bord antérieur du thalamus sur le second plan axial ;
- onzième point de repérage (BPT) : le bord postérieur du thalamus sur le second plan axial ;
- douzième point de repérage (CA) : la commissure antérieure ;
- treizième point de repérage (A) : le bord médial du troisième ventricule au point mi-commissural dit point « MCP » ;
- quatorzième point de repérage (B) : la hauteur du thalamus sur le plan sagittal passant par le treizième point de repérage (A) ;
- quinzième point de repérage (C) : le milieu du segment [AB] défini par les treizième et quatorzième points de repérage ;
- seizième point de repérage (D) : le bord antérieur du thalamus sur la droite parallèle à (CACP) et passant par le quinzième point de repérage (C) :
- dix-septième point de repérage ($P_{culm}$) : le bord supérieur du putamen sur le plan coronal passant par le cinquième point de repérage (PM1)
- dix-huitième point de repérage ($P_{lat}$) : le bord latéral du putamen sur le plan coronal passant par le cinquième point de repérage (PM1).

Le plan axial de référence (ou plan transverse) est le plan passant par le centre du repère CA-CP et les axes Ox et Oy. Les premier, second et troisième plans axiaux sont définis en relation avec le plan axial de référence.

Le premier plan axial correspond au plan parallèle au plan axial de référence passant par la coordonnée z(C)-5 mm (point se situant 5 mm en dessous de la coordonnée z du quinzième point de repérage et dont les coordonnées x et y sont égales à zéro).

Le second plan axial correspond au plan parallèle au plan axial de référence passant par la coordonnée z(C) (coordonnée z du quinzième point de repérage et dont les coordonnées x et y sont égales à zéro).

Le troisième plan axial correspond au plan parallèle au plan axial de référence passant la coordonnée z(C)-10 mm (10 mm en dessous de la coordonnée z du quinzième point de repérage et dont les coordonnées x et y sont égales à zéro).

Par plan sagittal, il faut comprendre tout plan confondu ou parallèle au plan formé par les axes Oy et Oz. En outre, le plan sagittal choisi dans le mode de réalisation passe par la coordonnée x du treizième point de repérage (A).

Par plan coronal, il faut comprendre tout plan confondu ou parallèle au plan formé par les axes Ox et Oz. En outre, le plan coronal choisi dans le mode de réalisation passe par la coordonnée y du cinquième point de repérage (PM1).

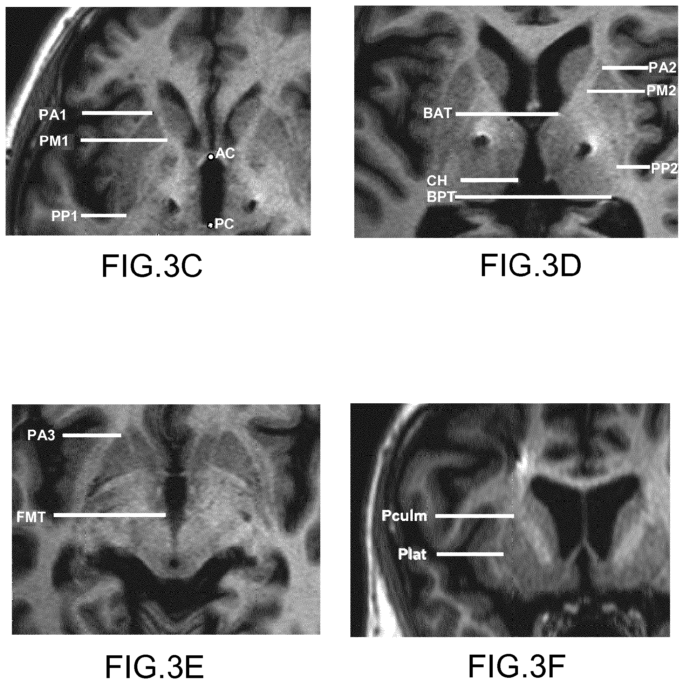

Les figures 3A à 3F représentent des images IRM vues selon différentes coupes. La figure 3A est une coupe axiale (plan axial de référence). La figure 3B est une coupe sagittale, selon le plan sagittal défini ci-dessus. La figure 3C est une coupe axiale (premier plan axial). La figure 3D est une coupe axiale (second plan axial). La figure 3E est une coupe axiale (troisième plan axial). La figure 3F est une coupe coronale, selon le plan coronal défini ci-dessus.

D'autres points de repérage peuvent être utilisés en fonction de l'intervention neurochirurgicale prévue. Ainsi, les points de ciblage dépendant de la pathologie à traiter, de manière générale, on peut choisir des points de repérage aptes à être corrélés avec lesdits points de ciblages et aisément repérables sur une imagerie classique, de type IRM.

Selon le mode de réalisation particulier, les points de repérage PR peuvent être représentés dans une matrice X où $X \in \mathbb{R}^{m \times 3}$, m représentant le nombre de points de repérage, m × 3 représentant l'ensemble des coordonnées de l'ensemble de ces points de repérage.

Chaque ligne de la matrice X représente un point de repérage donné. Chaque colonne représente une des 3 coordonnées.

En concaténant les coordonnées des points de repérage, la matrice X peut être reformée dans un vecteur $X \in \mathbb{R}^{3 \times m}$. Dans la suite de la description, on considèrera un tel vecteur X, qui comprend plusieurs (3 × m) éléments scalaires.

Chacun des 18 points de repérage est caractérisé par ses 3 coordonnées $x_{PR}$, $y_{PR}$, $z_{PR}$ dans le repère mathématique utilisé. Cela fait en tout 54 coordonnées.

En général, la détermination se fait par hémisphère : hémisphère droit (référencé « HD » dans la présente description) et hémisphère gauche (référencé « HG » dans la présente description). On a alors 18 points, soit 54 coordonnées dans HD et 18 points soit 54 coordonnées dans HG. Dans ce cas, m = 36 et on obtient en tout 108 coordonnées.

Ainsi, on peut soit construire une fonction de prédiction $F_G$ pour l'hémisphère gauche, qui permet de déterminer la cible à gauche, et une autre fonction de prédiction $F_D$ pour l'hémisphère droit qui permet de déterminer la cible à droite, soit construire une seule fonction F qui permet de déterminer les deux cibles (la cible à droite et la cible à gauche).

Points de ciblage (ou points de stimulation)

La cible (regroupant le ou les points de ciblage PC) est représentée dans un vecteur Y où $Y \in \mathbb{R}^p$. Un vecteur Y comprend plusieurs (p) éléments scalaires.

Le nombre de points de ciblage dans une cible est variable. La valeur p dépend du nombre de points de ciblage auquel on multiplie le nombre de coordonnées par point. Les coordonnées peuvent être les coordonnées cartésiennes $x_{PC}$, $y_{PC}$, $z_{PC}$ du point de ciblage.

Dans le cas d'une stimulation cérébrale profonde, les coordonnées peuvent comprendre en outre des coordonnées caractérisant la direction de l'électrode de stimulation en ce point. En connaissant un point de stimulation et la direction de l'électrode en ce point, on peut retrouver d'autres points de stimulation disposés sur une même électrode. Les points de stimulation sont aussi nommés « plots de stimulation ». Chaque électrode E1, E2 peut présenter par exemple 4 plots, comme représenté par exemple sur la figure 2. Le plot 0 peut représenter un point de ciblage PC1, PC2. Les patients peuvent ainsi être stimulés sur 1 à 2 plot(s) par hémisphère. Dans l'exemple de la figure 2, les plots 0 et 1 sont entièrement dans la cible, le plot 2 l'est partiellement et le plot 3 ne l'est pas. A titre d'exemple, chaque plot mesure 1,5 mm de long et deux plots sont espacés de 0,5 mm.

Si on recherche uniquement les coordonnées pour un point de ciblage, alors p=3 correspondant aux trois coordonnées $x_{PC}$, $y_{PC}$, $z_{PC}$ du point de ciblage dans le repère mathématique utilisé.

Si dans le cas d'une stimulation cérébrale profonde, on recherche en outre la direction de l'électrode pour un point de stimulation alors p = 6 correspondant à la cible $x_{PC}$, $y_{PC}$, $z_{PC}$ plus les coordonnées du vecteur directeur de l'électrode $v_{xPC}$, $V_{yPC}$, $V_{zPC}$ selon les trois axes du repère mathématique utilisé.

D'autres coordonnées peuvent être ajoutées en fonction de l'intervention neurochirurgicale prévue. Par exemple, pour un gammaknife ou un traitement par ultrasons focalisés, on peut adapter les coordonnées des points de ciblage.

Si on souhaite établir les coordonnées d'un point de ciblage d'une part pour l'hémisphère droit, et d'autre part pour l'hémisphère gauche, alors p = 6 (uniquement les coordonnées cartésiennes) ou p = 12 (incluant les coordonnées vectorielles).

Comme il peut donc y avoir un ou plusieurs points (ou plots) de ciblage dans une cible, la valeur p est égale au nombre de points multiplié par les coordonnées utilisées pour chaque point.

Base d'apprentissage

Pour un patient i (i = 1 ... n), on a un vecteur $X_i$ (de $3 \times$ m valeurs $x_i$) de points de repérage et un vecteur $Y_i$ (de p valeurs $y_i$) de points de ciblage. Les vecteurs $(X_i, Y_i)_{i=1 \,...\, n}$ extraits des n patients définissent l'ensemble de la base d'apprentissage.

## Etape d'apprentissage (construction d'une fonction Y = F(X))

A l'aide d'une méthode d'apprentissage statistique supervisé, la méthode selon l'invention construit une fonction de prédiction F telle que Y $\approx$ F (X).

La fonction de prédiction pourra être nommée simplement « fonction » dans la présente description.

La fonction F admet comme argument X ou une partie de X pour produire Y.

Pour un nouveau patient dont il faut déterminer la cible, il faut alors déterminer le vecteur X' des points de repérage de l'IRM, par exemple une IRM préopératoire réalisée avant la chirurgie.

La position Y' de la cible est ensuite déterminée à l'aide de la fonction F comme suit :

$$\widetilde{Y}' = F(\widetilde{X}')$$

Y̌' est la valeur obtenue pour la position Y' de la cible par la méthode selon l'invention.

Selon un mode de réalisation particulier, la méthode d'apprentissage statistique supervisé comprend l'utilisation de la méthode de régression ridge à noyaux dans un espace de Hilbert à noyau reproduisant.

La description qui suit décrit ce mode de réalisation particulier.

La régression ridge à noyau (« kernel ridge regression » en langue anglaise) est une méthode est relativement simple. Elle exploite en général l'ensemble de la base d'apprentissage et est adaptée à des « petites » bases d'apprentissage (nombre de patient (n) inférieur à 20) ou à des bases « moyennes » (nombre de patients (n) entre 20 et 100).

La fonction F est recherchée dans un espace de Hilbert ($\mathcal{H}, \langle.,.\rangle_{\mathcal{H}}$) en utilisant la méthode des moindres carrés avec un terme de régularisation $\lambda\|F\|_{\mathcal{H}}$ où $\lambda$ est un coefficient de régularisation. La régularisation assure que la norme $\|F\|_{\mathcal{H}} = \langle F, F\rangle_{\mathcal{H}}^{\frac{1}{2}}$ et par conséquent la fonction F ne dégénère pas.

Ainsi, l'espace de Hilbert choisi $\mathcal{H}$ est un espace de Hilbert à noyau reproduisant basé sur un noyau gaussien :

$$K(\mathbf{x}, \mathbf{y}) = e^{-\frac{|\mathbf{x}-\mathbf{y}|^2}{2\sigma^2}}, \qquad \forall\, \mathbf{x}, \mathbf{y} \in \mathbb{R}^{3\times m}$$

Le noyau est dit « reproduisant » sur l'espace $\mathcal{H}$ par la propriété donnée par l'équation (1) suivante :

$$F(\mathbf{x}) = \langle F(.), K(., \mathbf{x})\rangle_{\mathcal{H}} \qquad (1)$$

$F \in \mathcal{H}, \; \forall \mathbf{x} \in \mathbb{R}^{3\times m}$ où $\langle.,.\rangle_{\mathcal{H}}$ est le produit scalaire dans $\mathcal{H}$.

On considère dans l'espace de Hilbert $\mathcal{H}$ le problème des moindres carrés régularisé suivant : étant donnée une base d'apprentissage $\{(\mathbf{X_i}, y_i)\}_{i=1}^{n} \in (\mathbb{R}^{3\times m} \times \mathbb{R})^{n}$ où n est le nombre d'éléments dans la base, l'objectif est de résoudre l'équation (2) suivante :

$$\min_{f\in\mathcal{H}} \left\{ \frac{1}{n} \sum_{i=1}^{n} (F(\mathbf{X_i}) - y_i)^2 + \lambda\|F\|_{\mathcal{H}}^2 \right\} \qquad (2)$$

où $\lambda > 0$ est le coefficient de régularisation donné.

Le théorème de représentation de Riesz indique que la solution à l'équation (2) peut s'écrire comme :

$$F(x) = \sum_{i=1}^{n} \alpha_i K(x, \mathbf{X_i})$$

où $\alpha = (\alpha_i)_{i=1\ldots n} \in \mathbb{R}^n$ est une nouvelle inconnue.

En définissant la matrice de Gram $K = (K_{ij})_{i,j=1\ldots n}$ avec $K_{ij} = K(x_i, x_j)$, l'équation (2) à résoudre équivaut à résoudre l'équation (3) suivante :

$$\min_{\alpha\in\mathbb{R}^n} \left\{ \frac{1}{n} (K\alpha - y)^T (K\alpha - y) + \lambda\alpha^T K\alpha \right\} \qquad (3)$$

dont la solution est donnée simplement par l'équation (4):

$$\alpha = (K + \lambda nI)^{-1} y \qquad (4)$$

I étant la matrice identité dans $\mathbb{R}^n$.

Puisque la cible (regroupant le ou les points de ciblage) n'est pas définie par seulement une caractéristique scalaire mais par un vecteur Y de taille p contenant les coordonnées de chaque point de ciblage et/ou la direction d'une électrode de stimulation à positionner en chaque point (caractérisée par les coordonnées d'un vecteur dans les trois axes) et/ou d'autres coordonnées en fonction de l'intervention neurochirurgicale prévue, il s'agit de résoudre l'équation (5) suivante :

$$A = (K + \lambda nI)^{-1}Y \qquad (5)$$

I étant la matrice identité dans $\mathbb{R}^n$.

$A \in \mathbb{R}^{n \times p}$ et $Y \in \mathbb{R}^{n \times p}$.

Chaque colonne de A correspond au coefficient $\alpha$ pour une coordonnée donnée de la cible (entre 1 et p). Chaque colonne de Y correspond aux valeurs d'une coordonnée donnée de la cible (entre 1 et p).

Sachant que, comme indiqué plus haut, K est le noyau gaussien défini par la matrice de Gram :

$$K = (K_{i,j})_{i,j=1\ldots n} = K(x_i, x_j) = e^{-\frac{\left|(x_i)-(x_j)\right|^2}{2\sigma^2}} \qquad (6)$$

**Etape de reconstruction (nouveau patient)**

Une fois l'étape d'apprentissage terminée, c'est à dire la fonction F construite, on peut reconstruire une cible, c'est-à-dire déterminer le vecteur $\tilde{Y}$ des coordonnées du (ou des) point(s) de ciblage à partir d'un ensemble de points de repérage $\tilde{X}' \in \mathbb{R}^{3 \times m}$ déterminés par exemple à partir de l'IRM d'un patient, et ce en utilisant l'équation (7) suivante :

$$\tilde{Y}' = \tilde{K}A \qquad (7)$$

avec

$$\tilde{K}_j = K(\tilde{X}'; x_j),$$

j = 1 ... n

**Dans le cas d'ajout de données fonctionnelles dans la base,** les notations suivantes sont introduites.

Au moins un vecteur $\mathbf{X_i}$ prend en compte toutes les données anatomiques préopératoires comme les 18 points de repérage, mais aussi des données fonctionnelles comme les scores d'évaluation des symptômes de la pathologie. Le vecteur $\mathbf{X_i}$ sera donc de taille $3 \times m + M$, où M est le nombre de données fonctionnelles, par exemples des paramètres issus de l'évaluation des symptômes préopératoires (ou « scores préopératoires ») du patient.

Pour chaque patient i de la base, un vecteur postOpVector$_i$ est défini. Ce vecteur peut contenir par exemple des paramètres issus de l'évaluation des symptômes postopératoires (ou « scores postopératoires »), et/ou des paramètres électriques (fréquence, amplitude et durée) de stimulation.

La fonction à minimiser dans le cas de la prise en compte des données fonctionnelles est :

$$\min_{f \in \mathcal{H}} \left\{ \frac{1}{n} \sum_{i=1}^{n} (F(\mathbf{X_i}) - y_i)^2 \, W(\mathbf{postOpVector_i}) + \lambda \|F\|_{\mathcal{H}}^2 \right\} \qquad (8)$$

où W est la fonction poids et mesure la confiance dans les données d'un patient. Cette fonction poids peut être choisie complexe, ou simple. Un exemple de la fonction poids est l'inverse de l'amplitude de stimulation ou l'inverse de la quantité de courant injecté par seconde, ce qui correspond à l'inverse du produit des paramètres de stimulation.

Dans ce dernier cas :

$$W(\mathbf{postOpVector_i}) = \frac{1}{\text{fréquence} * \text{durée} * \text{amplitude}} \qquad (9)$$

Un autre exemple de fonction poids est l'inverse de la somme des scores postopératoires du patient qui reflète le fait que plus le score est faible plus l'électrode est bien placée.

La fonction poids W pourra aussi prendre une autre expression combinant les scores postopératoires et les paramètres électriques.

### Etape de sélection

La fonction obtenue en résolvant l'équation (5) n'inclut aucune sélection de points de repérages ou de coordonnées de ces points, en ce sens que les 54 coordonnées des 18 points de repère extraits de l'IRM (ou les 108 coordonnées des 36 points si on prend en compte les deux hémisphères du cerveau) sont utilisés lors des étapes d'apprentissage et de reconstruction. Or certaines coordonnées ne sont pas corrélées à la cible. Cela compromet la précision de détermination de la cible.

De manière préférentielle, la méthode comprend une étape de sélection afin d'utiliser uniquement les coordonnées des points de repérage qui sont corrélées à la cible.

L'étape de sélection peut comprendre l'utilisation d'un opérateur de projection $\pi \in \Pi(\mathbb{R}^{3 \times m})$ où $\Pi(\mathbb{R}^{3 \times m})$ est l'ensemble de tous les projecteurs dans $\mathbb{R}^{3 \times m}$.

La sélection des coordonnées des points de repérage optimaux combinée à la construction de la fonction de prédiction F est permise par la résolution de l'équation (10) suivante :

$$\min_{F \in \mathcal{H}, \pi \in \Pi(\mathbb{R}^{3 \times m})} \left\{ \frac{1}{n} \sum_{i=1}^{n} (F(\pi(\mathbf{X_i})) - y_i)^2 + \lambda \|F \circ \pi\|_{\mathcal{H}}^2 \right\} \qquad (10)$$

La sélection correspond à l'optimisation de l'opérateur de projection $\pi$. En pratique, ceci est effectué en calculant la sensibilité de la fonction à minimiser à chacune des caractéristiques (coordonnées des points de repérage) considérées.

Cela permet d'améliorer la précision de détermination de la cible.

### Etape d'optimisation des paramètres

La méthode comprend la construction d'une fonction F qui dépend d'un nombre de paramètres relativement réduit qui doivent être optimisés pour obtenir la fonction la plus précise possible, et ce, afin d'obtenir la cible la plus précise (et donc la plus efficace) : le coefficient de régularisation $\lambda$, la largeur du noyau gaussien $\sigma$, ainsi que l'opérateur de projection $\pi$.

L'optimisation de ces paramètres est résolue de manière itérative.

Les paramètres $\lambda$, $\sigma$ et $\pi$ sont optimisés en utilisant une procédure dite de « validation croisée » du type « leave-one-out ».

Le principe de la validation croisée du type « leave-one-out » est de construire une fonction de prédiction avec tous les exemples (ici les patients) d'une base d'apprentissage sauf un (patient) et d'évaluer la performance de la fonction construite sur l'exemple retiré de la base d'apprentissage. La procédure est reproduite en reproduisant cette procédure pour chacun des patients de l'ensemble de la base d'apprentissage.

Selon un mode de réalisation, on utilise pour les valeurs fixes de $\sigma$, $\lambda$ et de l'opérateur $\pi$, l'erreur de validation croisée « Leave One Out cross validation Error » (également nommée « LOOE »), qui est obtenue comme suit par la formule (11) ci-dessous :

$$LOOE\,(\sigma, \lambda, \pi) = \sum_{i=1}^{n} \frac{\left\|\mathbf{y_i} - \widetilde{\widetilde{Y}}_i\right\|^2}{\left(1 - S(i, i)\right)^2} \qquad (11)$$

où $S = K(K + \lambda I)^{-1}$ et $\widetilde{\widetilde{Y}} = S\mathbf{y}$.

La matrice K intègre l'opérateur de projection $\pi$ puisque:

$K = (K_{ij})_{i,j = 1 \dots n}$ avec $K_{i,j} = k(\pi(x_i), \pi(x_j))$.

Selon une première variante, les deux paramètres $\sigma$ et $\lambda$ et l'opérateur $\pi$ sont optimisés directement à l'aide d'un algorithme d'optimisation globale par stratégie d'évolution. Cet algorithme est basé sur la méthode CMA-ES (abréviation de « Covariance Matrix Adaptation-Evolution Strategy »), il permet de résoudre l'équation (12) ci-dessous :

$$(\sigma, \lambda, \pi) = \underset{\sigma\in\mathbb{R},\lambda\in\mathbb{R},\pi\in\Pi(\mathbb{R}^{3\times m})}{\operatorname{argmin}} \text{LOOE}\,(\sigma,\lambda,\pi) \qquad (12)$$

Selon cette première variante, la méthode comprend les étapes suivantes correspondant à une optimisation directe :

- Etape 1 : construction/initialisation de la base d'apprentissage

  ◦ Charger les données

  ◦ Définir les données d'entrée (points de repérage PR) $(X_i)_{i-1}^n$

  ◦ Définir les données de sortie (points de ciblage PC) $(Y_i)_{i=1}^n$
  ◦ Construire une fonction F préliminaire $F_P$

- Etape 2 : optimisation des paramètres

  ◦ Résoudre l'équation (12) ;

- Etape 3 : construction de la fonction F consolidée $F_C$ avec les paramètres $\lambda$, $\sigma$ et $\pi$ optimaux ($\pi$ intégrant les coordonnées optimales des points de repérage PR) ;
- Etape 4 : reconstruction pour un nouveau patient :

  ◦ Réalisation d'une IRM préopératoire (par exemple IRM 1.5T) ;
  ◦ Détermination des 18 points de repérage PR' visibles sur l'IRM ;
  ◦ Détermination des points de ciblage PC' (cible) en utilisant la fonction consolidée $F_C$ et les points de repérage PR'.

Selon cette première variante, l'étape de sélection et l'étape d'optimisation sont réalisées en même temps, la sélection se faisant par l'optimisation de l'opérateur de projection $\pi$.

Le problème de cette première variante de réalisation est la dimension de l'opérateur de projection $\pi$ qui est de 18 $\times$ 3 = 54, ou de 18 $\times$ 3 $\times$ 2 = 108 dans le cas où les deux hémisphères sont combinés pour la reconstruction de la cible. Dans ce cas, le nombre de paramètres à optimiser est trop grand dans le sens où le nombre de possibilités pour l'opérateur $\pi$ est $2^{54}$ ou $2^{108}$ suivant la taille de l'operateur en plus des deux paramètres $\lambda$ et $\sigma$.

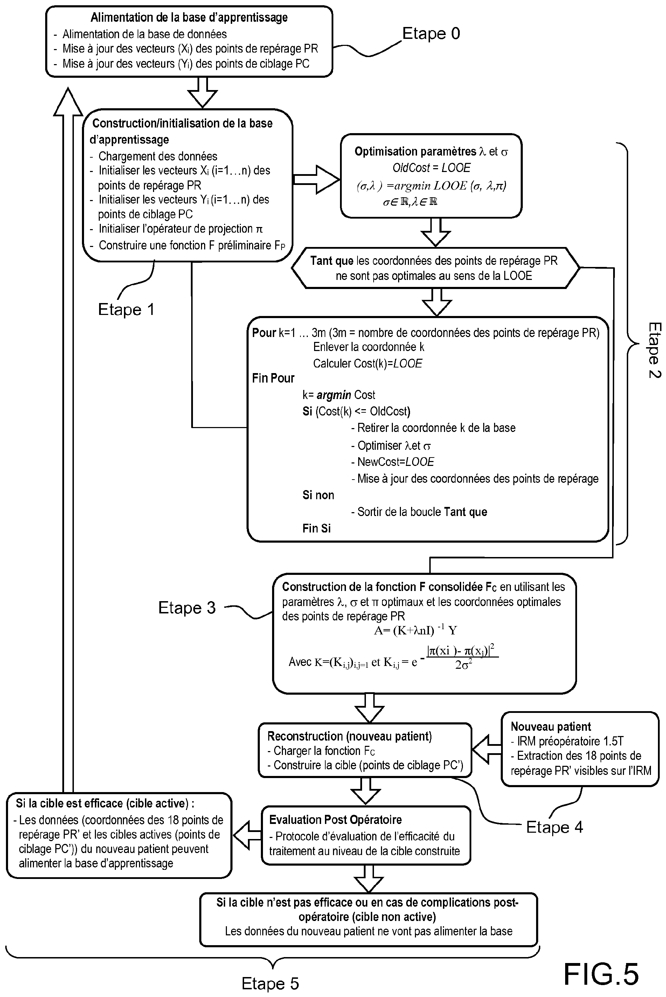

Selon une seconde variante décrite notamment en référence à la figure 5, les deux paramètres $\sigma$ et $\lambda$ et l'opérateur $\pi$ sont optimisés indirectement. On utilise une méthode itérative capable d'optimiser à la fois les paramètres $\lambda$ et $\sigma$ et l'opérateur de projection $\pi$

On résout dans un premier temps l'équation (13) suivante :

$$(\sigma, \lambda) = \underset{\sigma\in\mathbb{R},\lambda\in\mathbb{R}}{\operatorname{argmin}} \text{LOOE}\,(\sigma,\lambda,\pi) \qquad (13)$$

Ensuite, on teste la sensibilité de la fonction LOOE à la suppression de l'une des coordonnées (k) des points de repérage. Lorsqu'on supprime une $k^{ème}$ coordonnée, on obtient et on traite deux résultats possibles :

- si la LOOE augmente, cela signifie que les points de ciblage et la $k^{ème}$ coordonnée sont corrélés et qu'il faut conserver cette coordonnée ;
- si la LOOE reste constante ou diminue, cela signifie que les points cibles et la $k^{ème}$ coordonnée ne sont pas corrélés et qu'on peut potentiellement supprimer cette $k^{ème}$ coordonnée.

On supprime parmi les coordonnées potentiellement supprimables celle qui sont les moins corrélées aux cibles.

À chaque fois qu'une coordonnée est supprimée, l'opérateur de projection $\pi$ est mis à jour (avec une coordonnée en moins) et les paramètres $\sigma$ et $\lambda$ sont optimisés à l'aide de la fonction LOOE.

Cette procédure est répétée jusqu'à ce qu'aucune coordonnée ne doive être supprimée. Dans ce cas, toutes les coordonnées restantes sont nécessaires pour conserver une solution optimale au sens de l'erreur LOOE.

Selon cette seconde variante, la méthode comprend les étapes suivantes, correspondant à une optimisation indirecte :

- Etape 1 : construction/initialisation de la base d'apprentissage

  o Charger les données

  o Définir les données d'entrée (points de repérage PR) $(X_i)_{i-1}^n$

  o Définir les données de sortie (points de ciblage PC) $(Y_i)_{i=1}^n$

  o Initialiser l'opérateur de projection $\pi$

  ∘ Construire une fonction F préliminaire $F_P$ en considérant que $\pi$ est égal à la fonction identité

- Etape 2 : optimisation des paramètres

  o Réaliser une première optimisation de $\sigma$ et $\lambda$ avec l'équation (13) ;

  o Réaliser l'opération de boucle : Tant que les coordonnées présentes dans l'opérateur $\pi$ ne sont pas optimales (au sens de la LOOE) :

  - Pour chaque coordonnée k (k = 1 à 3 $\times$ m) des points de repérage :

    ▪ réaliser le calcul de la LOOE sans la coordonnée k ;
    ▪ si la LOOE reste constante ou diminue, alors on note que la coordonnée k de la base d'apprentissage est potentiellement supprimable ;
    ▪ sinon conserver la coordonnée k et passer à la coordonnée suivante k+1 ;

  - supprimer parmi les coordonnées potentiellement supprimables la coordonnée $k_{min}$ ; la coordonnée $k_{min}$ étant celle qui est la moins corrélée aux cibles au sens de la LOOE, en d'autres termes celle qui, si elle est prise en compte, dégrade le plus la LOOE ;
  - mettre à jour l'opérateur $\pi$ sans la coordonnée $k_{min}$ et refaire une optimisation de $\sigma$ et $\lambda$ avec l'équation (13).

  ∘ Fin de la boucle tant que.

- Etape 3 : construction de la fonction F consolidée $F_C$ avec les paramètres $\lambda$, $\sigma$ et l'opérateur $\pi$ optimaux (intégrant les coordonnées optimales des points de repérage PR) ;
- Etape 4 : reconstruction pour un nouveau patient

  o Réalisation d'une IRM préopératoire (par exemple IRM 1.5T) ;
  o Détermination des 18 points de repérage PR' visibles sur l'IRM ;
  o Détermination des points de ciblage PC' (cible) en utilisant la fonction consolidée $F_C$ et les points de repérage PR'.

Comme illustré dans la figure 5, d'autres étapes peuvent être ajoutées à la méthode :

- une étape 5 d'évaluation de l'efficacité de la stimulation (plus largement du traitement neurochirurgical) réalisée en la cible déterminée selon la méthode : cette étape est réalisée dans les mois suivant le traitement. On peut par exemple utiliser l'échelle UPDRS-3 pour la maladie de Parkinson (cibles STN et GPi) et l'échelle Fahn-Tolosa-Marin pour le tremblement essentiel (cible VIM) ;
- une étape 0 d'alimentation de la base d'apprentissage : la base peut en effet être alimentée à l'aide de nouveaux cas de patients dont le résultat clinique est jugé optimal selon l'évaluation post-opératoire (dont la cible a été déterminée ou non par la méthode selon l'invention).

L'étape 5 et/ou l'étape 0 permettent d'enrichir la base d'apprentissage avec les données de nouveaux patients pour lesquels le traitement a été évalué efficace.

D'autres méthodes d'apprentissage statistique supervisé peuvent être utilisées, par exemple une méthode de type machine à vecteurs de support (« Support Vector Machine » en langue anglaise dont l'acronyme est « SVM ») qui exploite non pas toute la base d'apprentissage mais seulement un sous-ensemble (les vecteurs de support), à l'inverse de la régression ridge à noyau. L'utilisation d'une telle méthode est possible lorsqu'on dispose d'une « grande » base d'apprentissage, c'est à dire lorsque le nombre de patients (n) dans la base est supérieur ou égal à 100. Dans ce cas, cela permet d'avoir une méthode moins onéreuse.

On peut également utiliser une méthode du type réseaux de neurones lorsqu'on dispose d'une grande base d'apprentissage.

Avantageusement, lorsque la base d'apprentissage est enrichie des données de nouveaux patients jusqu'à atteindre la taille critique de 100 patients, une méthode du type machine à vecteurs de support ou réseau de neurone peut être utilisée en tant que méthode d'apprentissage statistique supervisé pour construire la fonction F(X) = Y.

Avantageusement, on peut aussi introduire une étape de classification statistique (supervisée ou non supervisée) pour définir plusieurs classes dans la base d'apprentissage, chaque classe contenant un sous-ensemble de données de patients (en fonction par exemple de la pathologie, de l'âge, d'autres maladies ...) distinctes des données d'une autre classe. Ensuite pour chacune des classes, un metamodèle, c'est à dire une fonction de prédiction F est construit, par exemple comme décrit dans la seconde variante en référence à la figure 5.

Une classification statistique (supervisée ou non supervisée) peut également être mise en place en fonction des coordonnées des points de repérage des différents patients de la base. Dans ce dernier cas, les classes peuvent regrouper des patients ayant des coordonnées similaires, qui font que leurs cibles peuvent être également similaires.

Pour la construction de la cible pour un nouveau patient donné, il suffit d'identifier à quelle classe appartient le nouveau patient et d'utiliser la fonction F correspondant à cette classe pour prédire la cible. Cela peut permettre d'améliorer la précision de ciblage.

La classification statistique peut être réalisée en utilisant l'une parmi les méthodes suivantes :

- Régression logistique
- SVM
- Méthode de classification hiérarchique
- Réseaux de neurones
- Forêt d'arbres décisionnels

Toute autre méthode de classification statistique adaptée peut être utilisée.

Les différents modes présentés peuvent être combinés entre eux.

Tout ou partie des étapes suivantes : étape de construction et/ou d'initialisation de la base d'apprentissage, étape d'apprentissage, étape de sélection, étape d'optimisation des paramètres, étape de reconstruction, définition du repère mathématique et/ou des points de repérage décrits dans le mode de réalisation peuvent être utilisées pour toute application de la méthode autre que la stimulation chirurgicale profonde, de manière générale en préparation à tout traitement neurochirurgical nécessitant la détermination d'au moins une cible cérébrale précise.

En outre, la présente invention n'est pas limitée aux modes de réalisation précédemment décrits mais s'étend à tout mode de réalisation entrant dans la portée des revendications.

La méthode, le système et le programme d'ordinateur selon l'invention peuvent trouver des applications autres que la préparation à la stimulation cérébrale profonde. L'invention peut être utilisée dans une étape de préparation d'un traitement de type « gamaknife » afin de déterminer la zone d'application du faisceau ionisant. Elle peut également être utilisée dans une étape de préparation à un traitement par ultrasons focalisés de haute intensité (HIFU) afin de déterminer la zone d'application du faisceau d'ultrasons.

D'une manière générale, l'invention peut trouver une application pour la préparation de tout traitement neurochirurgical nécessitant la détermination d'au moins une cible cérébrale.

## Revendications

**1.** Méthode de détermination d'une cible cérébrale stéréotaxique comprenant au moins un point de ciblage (PC'), ladite méthode étant mise en œuvre au niveau de ladite cible pour une pathologie donnée sur un processeur en préparation à un traitement neurochirurgical de manière à améliorer le traitement neurochirurgical postérieur préalablement à un traitement neurochirurgical et comprenant les étapes suivantes :

- sélection d'une pluralité (n) de cas cliniques de patients pour lesquels le résultat mesuré suite au traitement réalisé pour ladite pathologie en au moins un point de ciblage (PC) est supérieur ou égal à un seuil, une imagerie postopératoire ayant été réalisée pour chacun desdits patients ;
- sélection d'un repère mathématique, de préférence un repère cartésien orthonormé ;
- traitement de ladite imagerie postopératoire de manière à déterminer pour chaque cas clinique sélectionné toute ou partie des (p) coordonnées $(x_{PC}, y_{PC}, z_{PC}, V_{xPC}, V_{yPC}, v_{zPC})$ du au moins un point de ciblage (PC) dans le repère sélectionné ;
- sélection d'une pluralité (m) de points de repérages (PR) du cerveau ;
- traitement de l'imagerie postopératoire de manière à déterminer pour chaque cas clinique sélectionné toute ou partie des $(3 \times m)$ coordonnées $(x_{PR}, y_{PR}, z_{PR})$ des points de repérage (PR) du cerveau ;

- constitution d'une base d'apprentissage comprenant les coordonnées déterminées des points de ciblage (PC) et les coordonnées déterminées des points de repérages (PR) de l'ensemble des (n) cas cliniques sélectionné ;
- détermination d'une fonction de prédiction (F) donnant les coordonnées d'au moins un point de ciblage (PC) en fonction des points de repérages (PR) en utilisant la base d'apprentissage et une méthode d'apprentissage statistique supervisé ;
- traitement d'une imagerie préopératoire d'un nouveau patient à traiter pour ladite pathologie de manière à déterminer toute ou partie des coordonnées ($x_{PR'}$, $y_{PR'}$, $z_{PR'}$) des points de repérages (PR') dudit nouveau patient;
- utilisation de la fonction de prédiction (F) de manière à obtenir les coordonnées d'au moins un point de ciblage (PC') pour ledit nouveau patient en fonction des coordonnées des points de repérages (PR') déterminés pour ledit nouveau patient.

2. Méthode selon la revendication 1, comprenant en outre une étape de :

- consolidation de la fonction de prédiction (F) en utilisant une méthode de validation croisée, ladite étape de consolidation aboutissant à une fonction de prédiction consolidée ($F_C$) donnant les coordonnées d'au moins un point de ciblage (PC) en fonction des points de repérage (PR) ;

l'étape d'utilisation de la fonction de prédiction (F) consistant en l'utilisation de la fonction de prédiction consolidée ($F_C$).

3. Méthode selon la revendication 1 ou 2, l'imagerie traitée pour déterminer les coordonnées des points de repérage (PR, PR') et/ou du au moins un point de ciblage (PC) étant au moins une image IRM, de préférence plusieurs images IRM.

4. Méthode selon l'une des revendications 1 à 3, la méthode d'apprentissage statistique supervisé comprenant l'utilisation d'une méthode de régression ridge à noyaux dans un espace de Hilbert à noyau reproduisant.

5. Méthode selon l'une des revendications 1 à 3, la méthode d'apprentissage statistique supervisé comprenant l'utilisation d'une méthode de type Machine à Vecteurs de Supports.

6. Méthode selon l'une des revendications 1 à 3, la méthode d'apprentissage statistique supervisé comprenant l'utilisation d'une méthode de type réseaux de neurones.

7. Méthode selon l'une des revendications 2 à 6, la méthode de validation croisée comprenant l'utilisation d'une méthode « leave-one-out cross-validation ».

8. Méthode selon l'une des revendications 2 à 6, la méthode de validation croisée comprenant l'utilisation d'une méthode « leave-k-out cross validation ».

9. Méthode selon l'une des revendications 1 à 8, le repère mathématique étant un repère cartésien orthonormé, la droite (CACP) passant par le bord supérieur et postérieur de la commissure antérieure (CA) et le bord inférieur et antérieur de la commissure postérieure (CP) formant l'axe Oy, le bord inférieur et antérieur de la commissure postérieure (CP) formant le centre du repère, et l'axe Oz étant la droite perpendiculaire à la droite (CACP) dans le plan inter-hémisphérique.

10. Méthode selon l'une des revendications 1 à 9, les points de repérage (PR) étant choisis parmi les dix-huit points suivants :

- le premier point de repérage (FMT) étant le faisceau mamillo-thalamique sur le troisième plan axial ;
- les deuxième, troisième et quatrième points de repérage (PA1, PA2, PA3) étant la pointe antérieure du putamen sur chacun des premier, second et troisième plans axiaux ;
- les cinquième et sixième points de repérage (PM1, PM2) étant la pointe médiale du putamen sur les premier et second plans axiaux ;
- les septième et huitième points de repérage (PP1, PP2) étant la pointe postérieure du putamen sur les premier et second plans axiaux ;
- le neuvième point de repérage (CH) étant la commissure habénulaire sur le second plan axial ;
- le dixième point de repérage (BAT) étant le bord antérieur du thalamus sur le second plan axial ;

17

- le onzième point de repérage (BPT) étant le bord postérieur du thalamus sur le second plan axial ;
- le douzième point de repérage (CA) étant la commissure antérieure ;
- le treizième point de repérage (A) étant le bord médial du troisième ventricule au point mi-commissural ;
- le quatorzième point de repérage (B) étant la hauteur du thalamus sur le plan sagittal passant par le treizième point de repérage (A) ;
- le quinzième point de repérage (C) étant le milieu du segment [AB] défini par les treizième et quatorzième points de repérage ;
- le seizième point de repérage (D) étant le bord antérieur du thalamus sur la droite parallèle à la droite (CACP) passant par le bord supérieur et postérieur de la commissure antérieure et le bord inférieur et antérieur de la commissure postérieure et passant par le quinzième point de repérage (C) ;
- le dix-septième point de repérage ($P_{culm}$) étant le bord supérieur du putamen sur le plan coronal passant par le cinquième point de repérage (PM1) ; et
- le dix-huitième point de repérage ($P_{lat}$) étant le bord latéral du putamen sur le plan coronal passant par le cinquième point de repérage (PM1).

11. Méthode selon l'une des revendications 1 à 10 comprenant en outre une étape supplémentaire d'ajout de données fonctionnelles dans la base d'apprentissage, lesdites données fonctionnelles étant aptes à ajouter au moins un indice du degré de confiance dans les points de ciblage (PC) et les points de repérages (PR) des cas cliniques.

12. Méthode selon l'une des revendications 1 à 11 mise en œuvre sur un processeur en préparation à une stimulation cérébrale profonde, à un gammaknife, ou à un traitement par ultrasons focalisés.

13. Système de traitement de données comprenant un processeur configuré pour mettre en œuvre tout ou partie des étapes de la méthode selon l'une des revendications 1 à 12.

14. Programme d'ordinateur comprenant des instructions qui, lorsque le programme est exécuté par un processeur, conduisent celui-ci à mettre en œuvre tout ou partie des étapes de la méthode selon l'une des revendications 1 à 12.

**Patentansprüche**

1. Verfahren zum Bestimmen eines stereotaktischen Hirntargets, umfassend mindestens einen Targeting-Punkt (PC'), wobei das Verfahren an dem Target für eine gegebene Pathologie auf einem Prozessor als Vorbereitung auf eine neurochirurgische Behandlung umgesetzt wird, um die neurochirurgische Nachbehandlung vor einer neurochirurgischen Behandlung zu verbessern, und die folgenden Schritte umfassend:

- Auswählen einer Vielzahl (n) von klinischen Fällen von Patienten, bei denen das Messergebnis nach der Behandlung, die für die Pathologie an mindestens einem Targeting-Punkt (PC) vorgenommen wird, größer als oder gleich einem Schwellenwert ist, wobei für jeden der Patienten eine postoperative Aufnahme erstellt worden ist;
- Auswählen eines mathematischen Koordinatensystems, vorzugsweise eines kartesischen orthonormierten Koordinatensystems;
- Verarbeiten der postoperativen Aufnahme, um für jeden ausgewählten klinischen Fall alle oder einen Teil der (p) Koordinaten ($x_{PC}$, $y_{PC}$, $z_{PC}$, $V_{xPC}$, $V_{yPC}$, $v_{zPC}$) des mindestens einen Targeting-Punktes (PC) in dem ausgewählten Koordinatensystem zu bestimmen;
- Auswählen einer Vielzahl (m) von Markierungspunkten (PR) des Hirns;
- Verarbeiten der postoperativen Aufnahme, um für jeden ausgewählten klinischen Fall alle oder einen Teil der (3 × m) Koordinaten ($x_{PR}$, $y_{PR}$, $z_{PR}$) der Markierungspunkte (PR) des Hirns zu bestimmen;
- Bilden einer Lerndatenbank, umfassend die bestimmten Koordinaten der Targeting-Punkte (PC) und die bestimmten Koordinaten der Markierungspunkte (PR) der Gesamtheit der (n) ausgewählten klinischen Fälle;
- unter Verwendung der Lerndatenbank und eines überwachten statistischen Lernverfahrens, Bestimmen einer Vorhersagefunktion (F), die die Koordinaten von mindestens einem Targeting-Punkt (PC) in Abhängigkeit der Markierungspunkte (PR) ergibt;
- Verarbeiten einer präoperativen Aufnahme eines neuen, wegen der Pathologie zu behandelnden Patienten, um alle oder einen Teil der Koordinaten ($x_{PR'}$, $y_{PR'}$, $z_{PR'}$) der Markierungspunkte (PR') des neuen Patienten zu bestimmen;
- Verwenden der Vorhersagefunktion (F), um die Koordinaten mindestens eines Targeting-Punktes (PC') für den neuen Patienten in Abhängigkeit der Koordinaten der für den neuen Patienten bestimmten Markierungspunkte (PR') zu erhalten.

2. Verfahren nach Anspruch 1, ferner umfassend einen Schritt zum:

- Konsolidieren der Vorhersagefunktion (F) unter Verwendung eines Kreuzvalidierungsverfahrens, wobei der Konsolidierungsschritt zu einer konsolidierten Vorhersagefunktion (F$_C$) führt, die die Koordinaten mindestens eines Targeting-Punktes (PC) in Abhängigkeit der Markierungspunkte (PR) ergibt;

wobei der Schritt zum Verwenden der Vorhersagefunktion (F) in der Verwendung der konsolidierten Vorhersagefunktion (F$_C$) besteht.

3. Verfahren nach Anspruch 1 oder 2, wobei es sich bei der verarbeiteten Aufnahme zum Bestimmen der Koordinaten der Markierungspunkte (PR, PR') und/oder des mindestens einen Targeting-Punktes (PC) um mindestens ein MRT-Bild, vorzugweise mehrere MRT-Bilder handelt.

4. Verfahren nach einem der Ansprüche 1 bis 3, wobei das überwachte statistische Lernverfahren die Verwendung eines Ridge-Kernregressionsverfahrens in einem kernreproduzierenden Hilbertraum umfasst.

5. Verfahren nach einem der Ansprüche 1 bis 3, wobei das überwachte statistische Lernverfahren die Verwendung eines Verfahrens vom Typ Support Vector Machine umfasst.

6. Verfahren nach einem der Ansprüche 1 bis 3, wobei das überwachte statistische Lernverfahren die Verwendung eines Verfahrens vom Typ neuronales Netz umfasst.

7. Verfahren nach einem der Ansprüche 2 bis 6, wobei das Kreuzvalidierungsverfahren die Verwendung eines Verfahrens der "Leave-One-Out-Kreuzvalidierung" umfasst.

8. Verfahren nach einem der Ansprüche 2 bis 6, wobei das Kreuzvalidierungsverfahren die Verwendung eines Verfahrens der "Leave-k-Out-Kreuzvalidierung" umfasst.

9. Verfahren nach einem der Ansprüche 1 bis 8, wobei das mathematische Koordinatensystem ein orthonormiertes kartesisches Koordinatensystem ist, wobei die Gerade (CACP) durch die obere und hintere Grenze der anterioren Kommissur (CA) hindurchgeht und die untere und vordere Grenze der posterioren Kommissur (CP) die Achse Oy bildet, wobei die untere und vordere Grenze der posterioren Kommissur (CP) die Mitte des Markierungspunktes bildet, und wobei die Achse Oz die Gerade ist, die senkrecht zur Geraden (CACP) in der Ebene der Zwischenhemisphäre steht.

10. Verfahren nach einem der Ansprüche 1 bis 9, wobei die Markierungspunkte (PR) aus den folgenden achtzehn Punkten ausgewählt sind:

- der erste Markierungspunkt (FMT) ist der Fasciculus mamillothalamicus auf der dritten axialen Ebene;
- der zweite, dritte und vierte Markierungspunkt (PA1, PA2, PA3) sind die vordere Spitze des Putamen auf jeder der ersten, zweiten und dritten axialen Ebene;
- der fünfte und sechste Markierungspunkt (PM1, PM2) sind die mittlere Spitze des Putamen auf der ersten und zweiten axialen Ebene;
- der siebte und achte Markierungspunkt (PP1, PP2) sind die hintere Spitze des Putamen auf der ersten und zweiten axialen Ebene;
- der neunte Markierungspunkt (CH) ist die Habenula-Kommissur auf der zweiten axialen Ebene;
- der zehnte Markierungspunkt (BAT) ist die vordere Grenze des Thalamus auf der zweiten axialen Ebene;
- der elfte Markierungspunkt (BPT) ist die hintere Grenze des Thalamus auf der zweiten axialen Ebene;
- der zwölfte Markierungspunkt (CA) ist die anteriore Kommissur;
- der dreizehnte Markierungspunkt (A) ist die mittlere Grenze des dritten Ventrikels am Punkt in der Mitte der Kommissur;
- der vierzehnte Markierungspunkt (B) ist die Höhe des Thalamus auf der Sagittalebene, die durch den dreizehnten Markierungspunkt (A) hindurchgeht;
- der fünfzehnte Markierungspunkt (C) ist die Mitte des Segments [AB], das durch den dreizehnten und vierzehnten Markierungspunkt definiert ist;
- der sechzehnte Markierungspunkt (D) ist die vordere Grenze des Thalamus auf der Geraden, die zur Geraden (CACP) parallel ist, die durch die obere und hintere Grenze der anterioren Kommissur und die untere und vordere Grenze der posterioren Kommissur hindurchgeht und durch den fünfzehnten Markierungspunkt (C) hindurchgeht;
- der siebzehnte Markierungspunkt ($P_{culm}$) ist die obere Grenze des Putamen auf der koronalen Ebene, die durch den fünften Markierungspunkt (PM1) hindurchgeht; und
- der achtzehnte Markierungspunkt ($P_{lat}$) ist die seitliche Grenze des Putamen auf der koronalen Ebene, die durch den fünften Markierungspunkt (PM1) hindurchgeht.

11. Verfahren nach einem der Ansprüche 1 bis 10, ferner umfassend einen zusätzlichen Schritt des Hinzufügens von funktionellen Daten in der Lerndatenbank, wobei die funktionellen Daten dazu geeignet sind, mindestens eine Indexziffer des Konfidenzgrads in den Targeting-Punkten (PC) und den Markierungspunkten (PR) der klinischen Fälle hinzuzufügen.

12. Verfahren nach einem der Ansprüche 1 bis 11, das auf einem Prozessor als Vorbereitung auf eine tiefe Hirnstimulation, ein Gamma-Knife oder eine Behandlung durch fokussierten Ultraschall umgesetzt wird.

13. Datenverarbeitungssystem, umfassend einen Prozessor, der konfiguriert ist, um alle oder einen Teil der Schritte des Verfahrens nach einem der Ansprüche 1 bis 12 umzusetzen.

14. Computerprogramm, umfassend Befehle, die bei Ausführen des Programms durch einen Prozessor diesen veranlassen, alle oder einen Teil der Schritte des Verfahrens nach einem der Ansprüche 1 bis 12 umzusetzen.

**Claims**

1. A method for determining a stereotactic brain target comprising at least one target point (PC'), said method being implemented at said target for a given pathology on a processor as a preparation to a neurosurgical treatment so as to improve the subsequent neurosurgical treatment prior to a neurosurgical treatment and comprising the following steps:

- selecting a plurality (n) of clinical cases of patients for whom the result measured following the treatment performed for said pathology at at least one target point (PC) is greater than or equal to a threshold, postoperative imaging having been performed for each of said patients;
- selecting a mathematical coordinate system, preferably an orthonormal Cartesian coordinate system;
- processing said postoperative imaging so as to determine, for each selected clinical case, all or some of the (p) coordinates ($x_{PC}$, $y_{PC}$, $z_{PC}$, $v_{xPC}$, $V_{yPC}$, $v_{zPC}$) of the at least one target point (PC) in the selected coordinate system;
- selecting a plurality (m) of marker points (PR) of the brain;
- processing the postoperative imaging so as to determine, for each selected clinical case, all or some of the (3 × m) coordinates ($x_{PR}$, $y_{PR}$, $z_{PR}$) of the marker points (PR) of the brain;
- creating a learning database comprising the determined coordinates of the target points (PC) and the determined coordinates of the marker points (PR) of all of the (n) selected clinical cases;
- determining a prediction function (F) giving the coordinates of at least one target point (PC) according to the marker points (PR) by using the learning database and a supervised statistical learning method;
- processing preoperative imaging of a new patient to be treated for said pathology so as to determine all or some of the coordinates ($x_{PR'}$, $y_{PR'}$, $z_{PR'}$) of the marker points (PR') of said new patient;
- using the prediction function (F) so as to obtain the coordinates of at least one target point (PC') for said new patient according to the coordinates of the marker points (PR') determined for said new patient.

2. The method according to claim 1, further comprising a step of:

- consolidating the prediction function (F) using a cross-validation method, said consolidating step resulting in a consolidated prediction function ($F_C$) giving the coordinates of at least one target point (PC) according to the marker points (PR);

the step of using the prediction function (F) consisting in using the consolidated prediction function ($F_C$).

3. The method according to claim 1 or 2, the imaging processed to determine the coordinates of the marker points (PR, PR') and/or of the at least one target point (PC) being at least one MRI image, preferably several MRI images.

4.   The method according to one of claims 1 to 3, the supervised statistical learning method comprising the use of a kernel ridge regression method in a reproducing kernel Hilbert space.

5.   The method according to one of claims 1 to 3, the supervised statistical learning method comprising the use of a method of support-vector machine type.

6.   The method according to one of claims 1 to 3, the supervised statistical learning method comprising the use of a method of neural network type.

7.   The method according to one of claims 2 to 6, the cross-validation method comprising the use of a "leave-one-out cross-validation" method.

8.   The method according to one of claims 2 to 6, the cross-validation method comprising the use of a "leave-k-out cross-validation" method.

9.   The method according to one of claims 1 to 8, the mathematical coordinate system being an orthonormal Cartesian coordinate system, the straight line (CACP) passing through the superior and posterior edge of the anterior commissure (CA) and the inferior and anterior edge of the posterior commissure (CP) forming the Oy axis, the inferior and anterior edge of the posterior commissure (CP) forming the centre of the coordinate system, and the Oz axis being the straight line perpendicular to the straight line (CACP) in the interhemispheric plane.

10.  The method according to one of claims 1 to 9, the marker points (PR) being chosen from among the following eighteen points:

- the first marker point (FMT) being the mammillothalamic fasciculus on the third axial plane;
- the second, third and fourth marker points (PA1, PA2, PA3) being the anterior point of the putamen on each of the first, second and third axial planes;
- the fifth and sixth marker points (PM1, PM2) being the medial tip of the putamen on the first and second axial planes;
- the seventh and eighth marker points (PP1, PP2) being the posterior tip of the putamen on the first and second axial planes;
- the ninth marker point (CH) being the habenular commissure on the second axial plane;
- the tenth marker point (BAT) being the anterior edge of the thalamus on the second axial plane;
- the eleventh marker point (BPT) being the posterior edge of the thalamus on the second axial plane;
- the twelfth marker point (CA) being the anterior commissure;
- the thirteenth marker point (A) being the medial edge of the third ventricle at the mid-commissural point;
- the fourteenth marker point (B) being the height of the thalamus on the sagittal plane passing through the thirteenth marker point (A);
- the fifteenth marker point (C) being the midpoint of the segment [AB] defined by the thirteenth and fourteenth marker points;
- the sixteenth marker point (D) being the anterior edge of the thalamus on the straight line parallel to the straight line (CACP) passing through the superior and posterior edge of the anterior commissure and the inferior and anterior edge of the posterior commissure and passing through the fifteenth marker point (C);
- the seventeenth marker point ($P_{culm}$) being the superior edge of the putamen on the coronal plane passing through the fifth marker point (PM1); and
- the eighteenth marker point ($P_{lat}$) being the lateral edge of the putamen on the coronal plane passing through the fifth marker point (PM1).

11.  The method according to one of claims 1 to 10, further comprising an additional step of adding functional data to the learning database, said functional data being able to add at least one indicator of the degree of confidence in the target points (PC) and the marker points (PR) of the clinical cases.

12.  The method according to one of claims 1 to 11, implemented on a processor as a preparation to deep brain stimulation, Gamma Knife, or focused ultrasound treatment.

13.  A data processing system comprising a processor configured to implement all or some of the steps of the method according to one of claims 1 to 12.

14. A computer program comprising instructions which, when the program is executed by a processor, cause it to implement all or some of the steps of the method according to one of claims 1 to 12.

FIG.1

FIG.2

FIG.3A

FIG.3B

FIG.3C

FIG.3D

FIG.3E

FIG.3F

FIG.4A

FIG.4B

FIG.4C

FIG.4D

**Alimentation de la base d'apprentissage**
- Alimentation de la base de données
- Mise à jour des vecteurs ($X_i$) des points de repérage PR
- Mise à jour des vecteurs ($Y_i$) des points de ciblage PC

Etape 0

**Construction/initialisation de la base d'apprentissage**
- Chargement des données
- Initialiser les vecteurs $X_i$ (i=1…n) des points de repérage PR
- Initialiser les vecteurs $Y_i$ (i=1…n) des points de ciblage PC
- Initialiser l'opérateur de projection $\pi$
- Construire une fonction F préliminaire $F_P$

Etape 1

**Optimisation paramètres $\lambda$ et $\sigma$**
$OldCost = LOOE$
$(\sigma,\lambda) = argmin\ LOOE\ (\sigma,\ \lambda,\pi)$
$\sigma \in \mathbb{R}, \lambda \in \mathbb{R}$

**Tant que** les coordonnées des points de repérage PR ne sont pas optimales au sens de la LOOE

**Pour** k=1 … 3m (3m = nombre de coordonnées des points de repérage PR)
    Enlever la coordonnée k
    Calculer Cost(k)=$LOOE$
**Fin Pour**

    k= **argmin** Cost
    **Si** (Cost(k) <= OldCost)
        - Retirer la coordonnée k de la base
        - Optimiser $\lambda$ et $\sigma$
        - NewCost=$LOOE$
        - Mise à jour des coordonnées des points de repérage
    **Si non**
        - Sortir de la boucle **Tant que**
    **Fin Si**

Etape 2

**Construction de la fonction F consolidée $F_C$ en utilisant les paramètres $\lambda$, $\sigma$ et $\pi$ optimaux et les coordonnées optimales des points de repérage PR**
$$A = (K+\lambda nI)^{-1} Y$$
Avec $K=(K_{i,j})_{i,j=1}$ et $K_{i,j} = e^{-\frac{|\pi(x_i) - \pi(x_j)|^2}{2\sigma^2}}$

Etape 3

**Reconstruction (nouveau patient)**
- Charger la fonction $F_C$
- Construire la cible (points de ciblage PC')

**Nouveau patient**
- IRM préopératoire 1.5T
- Extraction des 18 points de repérage PR' visibles sur l'IRM

Etape 4

**Evaluation Post Opératoire**
- Protocole d'évaluation de l'efficacité du traitement au niveau de la cible construite

**Si la cible est efficace (cible active) :**
- Les données (coordonnées des 18 points de repérage PR' et les cibles actives (points de ciblage PC')) du nouveau patient peuvent alimenter la base d'apprentissage

**Si la cible n'est pas efficace ou en cas de complications post-opératoire (cible non active)**
Les données du nouveau patient ne vont pas alimenter la base

Etape 5

**FIG.5**